(19) Europäisches Patentamt
European Patent Office
Office européen des brevets

(11) Numéro de publication: **0 133 406**
**B1**

# (12) FASCICULE DE BREVET EUROPEEN

(45) Date de publication du fascicule du brevet: **22.04.87**

(21) Numéro de dépôt: **84401616.2**

(22) Date de dépôt: **02.08.84**

(51) Int. Cl.[4]: **C 07 C 121/75**, C 07 C 121/48, C 07 D 213/64, C 07 D 233/74, C 07 D 307/45, A 01 N 53/00, A 23 K 1/16, A 61 K 31/275, A 61 K 31/33

**(54) Nouveaux dérivés de l'acide cyclopropane carboxylique, leur préparation, leur application à la lutte contre les parasites et les compositions les renfermant.**

(30) Priorité: **04.08.83 FR 8312858**
**19.01.84 FR 8400798**

(43) Date de publication de la demande:
**20.02.85 Bulletin 85/8**

(45) Mention de la délivrance du brevet:
**22.04.87 Bulletin 87/17**

(84) Etats contractants désignés:
**CH DE FR GB IT LI NL**

(56) Documents cités:
**GB - A - 2 099 810**

**Le dossier contient des informations techniques présentées postérieurement au dépôt de la demande et ne figurant pas dans le présent fascicule.**

(73) Titulaire: **ROUSSEL-UCLAF, 35, boulevard des Invalides, F-75007 Paris (FR)**

(72) Inventeur: **Tessier, Jean, 30, rue Jean Moulin, F-94300-Vincennes (FR)**
Inventeur: **Teche, André, 15, rue Godot de Mauroy, F-75009-Paris (FR)**
Inventeur: **Girault, Pierre, 4, rue des Abbesses, F-75018-Paris (FR)**

(74) Mandataire: **Fritel, Hubert et al, Département des Brevets ROUSSEL UCLAF B.P. no 9, F-93230 Romainville (FR)**

EP 0 133 406 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne de nouveaux dérivés de l'acide cyclopropane carboxylique, leur procédé de préparation, leur application à la lutte contre les parasites et les compositions les renfermant.

Le brevet anglais 2099810 a pour objet des composés de formule (A)

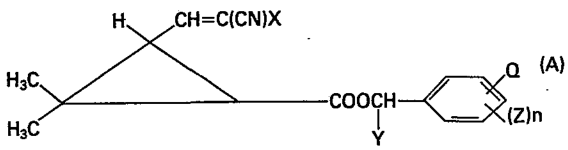

(A)

dans laquelle X est un atome de fluor de chlore ou de brome, Y est un atome d'hydrogène, un radical cyano ou un radical éthynyle, Z est un atome d'halogène, n est un nombre compris entre 0 et 4, Q est un atome d'hydrogène, d'halogène ou un radical phénoxy.

On vient de découvrir certains composés apparentés aux composés de formule A dans laquelle X représente un atome de fluor, et la copule cyclopropanique est de structure 1R cis.

L'invention a pour objet sous toutes leurs formes isomères possibles ou sous forme de mélanges de ces isomères, les composés de formule (I):

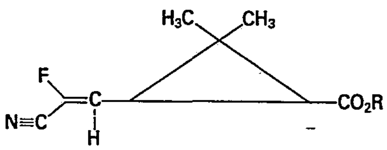

(I)

dans laquelle la copule acide cyclopropanique est de structure 1R cis, la géométrie de la double liaison portée par le carbone en 3 peut être de structure E ou Z et R représente un radical alcoyle linéaire, ramifié ou cyclisé, saturé ou insaturé, renfermant de 2 à 12 atomes de carbone ou un radical choisi dans le groupe des radicaux suivants:

les radicaux

-CH-Ar
 |
 Z

dans lesquels Z représente un radical éthynyle, méthyle ou cyano et Ar represente un radical

$-C_6H_5$
$-C_6F_5$

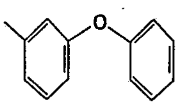, 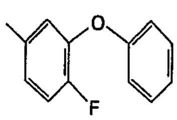,

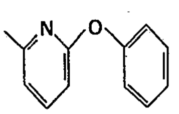, 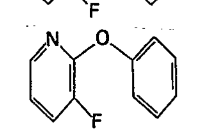

et 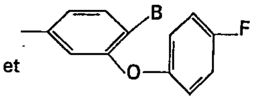

dans lequel B représente un atome d'hydrogène ou de fluor.

Lorsque R représente un radical alcoyle linéaire, ramifié ou cyclisé, saturé ou insaturé, il s'agit de préférence du radical éthyle, propyle linéaire ou ramifié, butyle linéaire ou ramifié, pentyle linéaire ou ramifié, hexyle, linéaire ou ramifié, cyclopropyle, cyclobutyle, cyclopentyle, cyclohexyle, 1-propényle, 1-butynyle, 1,3-butédiényle, 1-pentényle, 1-cyclobutynyle, 1-cyclopentadiényle, 1-cyclohexényle.

L'invention a tout spécialement pour objet les composés de formule (I) dans laquelle la géométrie de la double liaison portée par le carbone en 3 du cyclopropane est de structure Z.

Parmi les composés de l'invention, on peut citer tout particulièrement les composés pour lesquels R représente un radical

-CH-Ar,
 |
 Z

dans lequel Z représente un radical -C≡CH, $CH_3$ ou un radical -C≡N et Ar représente un radical choisi dans le groupe des radicaux

$-C_6H_5$
$-C_6F_5$

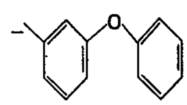 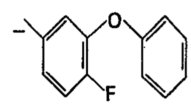

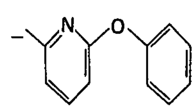, 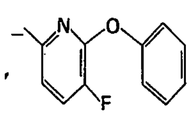

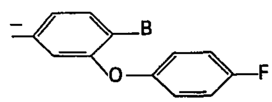

dans lequel B représente un atome d'hydrogène ou de fluor, et notamment les composés pour lesquels Ar représente un radical α-cyano-3-phénoxybenzyle.

Parmi les composés de l'invention, on peut encore citer les composés répondant à la formule (I), dans laquelle R représente un radical

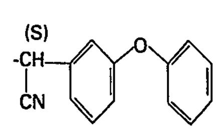 ou 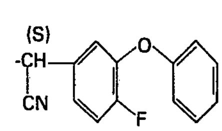

Parmi les composés de l'invention, on peut citer tout particulièrement les composés préparés ci-après dans la partie expérimentale et notamment:

– le 1R-cis-2,2-diméthyl-3-[ΔE-2-fluoro-2-cyanoéthényl]cyclopropane carboxylate de (S)α-cyano-3-phénoxybenzyle et l'isomère ΔZ correspondant;

– le 1R-cis-2,2-diméthyl-3-[ΔE-2-fluoro-2-cyanoéthényl]cyclopropane carboxylate de (S)α-cyano-3-phénoxy-4-fluorobenzyle et l'isomère ΔZ correspondant;

– le 1R-cis-2,2-diméthyl-3-[ΔE-2-fluoro-2-cyanoéthényl]cyclopropane carboxylate de (R,S)-cyano-2-(6-phénoxypyridyl)méthyle et l'isomère ΔZ correspondant.

Les composés de formule (I) présente d'intéressantes propriétés qui permettent leur utilisation dans la lutte contre les parasites. Il peut s'agir par exemple de la lutte contre les parasites des végétaux, les parasites des locaux et les parasites des animaux à sang chaud. C'est ainsi que l'on peut utiliser les produits de l'invention pour lutter contre les insectes, les nématodes et les acariens parasites des végétaux et des animaux.

L'invention a donc aussi pour objet l'application des composés de formule (I) tels que définis précédemment à la lutte contre les parasites des végétaux, les parasites des locaux.

Les produits de formule (I) peuvent être utilisés notamment pour lutter contre les insectes dans le domaine agricole, pour lutter par exemple contre les pucerons, les larves de lépidoptères et les coléoptères. Ils sont utilisés à des doses comprises entre 10 g et 300 g de matière active à l'hectare.

Les produits de formule (I) peuvent également être utilisés pour lutter contre les insectes dans les locaux, pour lutter notamment contre les mouches, les moustiques et les blattes.

Les produits de formule (I) peuvent aussi être utilisés pour lutter contre les acariens et les nématodes parasites des végétaux.

Les composés de formule (I) peuvent encore être utilisés pour lutter contre les acariens parasites des animaux, pour lutter par exemple contre les tiques et notamment les tiques de l'espèce Boophilus, ceux de l'espèce Hyalomnia, ceux de l'espèce Amblyomnia et ceux de l'espèce Rhipicephalus ou pour lutter contre toutes sortes de gales et notamment la gale sarcoptique, la gale psoroptique et la gale chorioptique. Ils peuvent aussi être utilisés contre les poux et les helminthes.

L'invention a donc également pour objet les compositions destinées à la lutte contre les parasites des végétaux, les parasites des locaux et les parasites des animaux à sang chaud, caractérisées en ce qu'elles renferment comme principe actif au moins un des produits de formule générale (I).

L'invention a notamment pour objet les compositions insecticides renfermant comme principe actif au moins l'un des produits de formule générale (I).

Parmi les compositions notamment insecticides préférées de l'invention, on peut citer tout spécialement les compositions renfermant les composés décrits dans les exemples et notamment

– le (1R-cis)-2,2-diméthyl-3-[(ΔE)-2-fluoro-2-cyanoéthényl]cyclopropane carboxylate de (S)-α-cyano-3-phénoxybenzyle et l'isomère ΔZ correspondant;

– le (1R-cis)-2,2-diméthyl-3-[(ΔE)-2-fluoro-2-cyanoéthényl]cyclopropane carboxylate de (S)-α-cyano-3-phénoxy-4-fluorobenzyle et l'isomère ΔZ correspondant;

– le (1R-cis)-2,2-diméthyl-3-[(ΔE)-2-fluoro-2-cyanoéthényl]cyclopropane carboxylate de (R,S)-cyano-2-(6-phénoxypyridyl)-méthyle et l'isomère ΔZ correspondant.

Ces compositions sont préparées selon les procédés usuels de l'industrie agrochimique. Elles peuvent être additionnées éventuellement d'un ou plusieurs autres agents pesticides. Ces compositions peuvent se présenter sous forme de poudres, granulés, suspensions, émulsions, solutions, solutions pour aérosols, bandes combustibles, appâts ou autres préparations employés classiquement pour l'utilisation de ce genre de composés.

Outre le principe actif, ces compositions contiennent, en général, un véhicule et/ou un agent tensio-actif, non ionique, assurant, en outre, une dispersion uniforme des substances constitutives du mélange. Le véhicule utilisé peut être un liquide, tel que l'eau, l'alcool, les hydrocarbures ou autres solvants organiques, une huile minérale, animale ou végétale, une poudre telle que le talc, les argiles, les silicates, le Kieselguhr ou un solide combustible.

Les compositions insecticides selon l'invention contiennent de préférence de 0,005% à 10% en poids de matière active.

Selon un mode opératoire avantageux, pour un usage dans les locaux, les compositions selon l'invention sont utilisées sous forme de compositions fumigantes.

Les compositions selon l'invention peuvent alors être avantageusement constituées, pour la partie non active, d'un serpentin combustible, ou encore d'un substrat fibreux incombustible. Dans ce dernier cas, le fumigant obtenu après incorporation de la matière active est placé sur un appareil chauffant.

Dans le cas où l'on utilise un serpentin insecticide, le support inerte peut être, par exemple, composé de marc de pyrèthre, poudre de Tabu (ou poudre de feuilles Machilus Thumbergii), poudre de tige de pyrèthre, poudre de feuille de cèdre, poudre de bois (telle que de la sciure de pin), amidon et poudre de coque de noix de coco.

La dose de matière active peut alors être, par exemple, de 0,03 à 1% en poids.

Dans le cas où l'on utilise un support fibreux incombustible, la dose de matière active peut alors être, par exemple, de 0,03 à 25% en poids.

Les compositions selon l'invention pour un usage dans les locaux peuvent aussi être obtenues en préparant une huile pulvérisable à base de principe actif, cette huile imbibant la mèche d'une lampe et étant alors soumise à la combustion.

La concentration du principe actif incorporé à l'huile est, de préférence, de 0,03 à 25% en poids.

L'invention a aussi notamment pour objet les compositions acaricides renfermant comme principe actif au moins l'un des produits de formule

générale (I) ainsi que les compositions nématicides renfermant comme principe actif au moins l'un des produits de formule générale (I).

Les compositions acaricides et nématicides peuvent se présenter notamment sous forme de poudres, granulés, suspensions, émulsions, solutions.

Les compositions acaricides et nématicides peuvent être additionnées éventuellement d'un ou plusieurs autres agents pesticides.

Pour l'usage acaricide, on utilise de préférence des poudres mouillables, pour pulvérisation foliaire, contenant de 1 à 80% de principe actif (ou des liquides pour pulvérisation foliaire contenant de 1 à 500 g/l de principe actif. On peut également employer des poudres pour poudrage foliaire contenant de 0,05 à 3% de matière active.

Pour l'usage nématicide, on utilise de préférence des liquides pour traitement des sols contenant de 300 à 500 g/l de principe actif.

Les composés acaricides et nématicides selon l'invention sont utilisés, de préférence, à des doses comprises entre 1 et 100 g de matière active à l'hectare.

L'invention a également pour objet les compositions acaricides contre les parasites des animaux à sang chaud, notamment contre les tiques et les gales, caractérisées en ce qu'elles renferment comme principe actif au moins un produit de formule générale I.

Lorsqu'il s'agit de lutter contre les parasites des animaux, les compositions peuvent être utilisées sous forme de spray, de bains, ou encore selon la méthode «pour-on».

Lorsqu'on utilise la méthode pour-on, on utilise de préférence des solutions renfermant de 0,5 à 4 g de principe actif pour 100 $cm^3$ de solution.

Lorsqu'il s'agit de lutter contre les acariens parasites des animaux, on peut incorporer les produits de l'invention dans des compositions alimentaires en association avec un mélange nutritif adapté à l'alimentation animale. Le mélange nutritiel peut varier selon l'espèce animale; il peut renfermer des céréales, des sucres et des grains, des tourteaux de soja, d'arachide et de tournesol, des farines d'origine animale, par exemple, des farines de poissons, des acides aminés de synthèse, des sels minéraux, des vitamines et des antioxydants.

L'invention a donc également pour objet les compositions destinées à l'alimentation animale renfermant comme principe actif au moins l'un des produits de formule générale (I).

L'invention a également pour objet les associations douées d'activité insecticide, acaricide ou nématicide, caractérisées en ce qu'elles contiennent comme matière active, d'une part un au moins des produits définis ci-dessus et d'autre part, un au moins des esters pyréthrinoïdes choisis dans le groupe constitué par les esters d'alléthrolone, d'alcool 3,4,5,6-tétrahydrophthalimidométhylique, d'alcool 5-benzyl-3-furyl-methylique, d'alcool 3-phénoxy-benzylique et d'alcools – cyano-3-phénoxy-benzyliques des acides chrysanthémiques, par les esters d'alcool 5-benzyl-3-furylméthylique des acides 2,2-diméthyl-3-(2-oxo-3-tétrahydrothiophénylidène-méthyl)-cyclopropane-1-carboxyliques, par les esters d'alcool 3-phénoxy-benzylique et d'alcool α-cyano-3-phénoxy, benzyliques des acides 2,2-diméthyl-3-(2,2-dichlorovinyl)-cyclopropane-1-carboxyliques, par les esters d'alcools α-cyano-3-phénoxy-benzyliques d'acides 2,2-diméthyl-3-(2,2-dibromovinyl)-cyclopropane-1-carboxyliques, par les esters d'alcool 3-phénoxy-benzylique des acides 2-parachlorphényl-2-isopropyl-acétiques, par les esters d'alléthrolone, d'alcool 3,4,5,6-tétrahydrophthalimidométhylique, d'alcool 5-benzyl-3-furyl-méthylique, d'alcool 3-phénoxy-benzylique et d'alcools α-cyano-3-phénoxy-benzyliques des acides 2,2-diméthyl-3-(1,2-2,2-tétrahaloéthyl)-cyclopropane-1-carboxyliques, dans lesquels «halo» représente un atome de fluor, de chlore ou de brome, étant entendu que les copules acides et alcools des esters pyréthrinoïdes ci-dessus peuvent exister sous toutes leurs formes stéréoisomères possibles.

L'invention a aussi pour objet les compositions ou associations définis précédemment, caractérisées en ce qu'elles renferment, en outre, un synergiste des pyréthrinoïdes.

Comme synergistes classiques utilisés en pareil cas, on peut citer le 1-(2,5,8-trioxadodécyl)-2-propyl-4,5-méthylène-dioxy-benzène (ou butoxyde de pipéronyle) ou le N-(2-éthylheptyl)-bicyclo[2,2-1]5-heptène-2,3-dicarboximide ou le pipéronyl-bis-2-(2n-butoxyéthoxy)éthyl-acétal (ou tropital).

L'invention a également pour objet un procédé de préparation des composés de formule (I) tels que définis précédemment, caractérisé en ce que l'on soumet selon la réaction de Wittig un composé de formule (I):

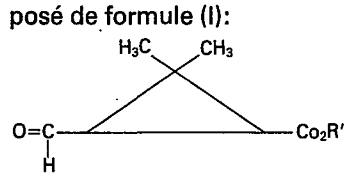 (II)

dans laquelle R' représente un atome d'hydrogène ou R, R conservant la même signification que précédemment, à l'action d'un composé de formule (III):

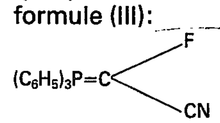 (III)

obtenir un composé correspondant que l'on soumet à l'action d'un agent d'estérification si R' représente un atome d'hydrogène, ou que l'on soumet, si désiré, à l'action d'un agent de clivage de la fonction ester, puis à l'action d'un agent d'estérification.

Les composés de formule (II) et de formule (III) sont des produits connus. Lorsque R représente un atome d'hydrogène et lorsque la configuration et cis, les composés de formule (II) peuvent se présenter sous la forme lactonique.

Les produits de formule (III) peuvent être préparés selon le procédé décrit dans Helv. Chem. Acta. Vol. 60 (1977) p. 585.

La réaction de Wittig utilisée généralement pour préparer les produits de formule (I) fournit des composés de formule (I) dont la géométrie est E + Z.

Les isomères au niveau de la double liaison peuvent être séparés, si désiré, par des méthodes physiques telles que la chromatographie, soit au niveau des acides, soit au niveau des esters d'alcoyle, soit au niveau des autres esters.

L'agent de clivage du groupement $CO_2R$ est de préférence la chaleur utilisée avec un agent d'hydrolyse acide. Comme agent d'hydrolyse acide, on peut utiliser l'acide p-toluène-sulfonique.

L'estérification peut être effectuée en présence d'une base tertiaire, telle que la pyridine. Cette estérification peut être effectuée avantageusement en présence d'un mélange de dicyclohexylcarbodiimide et de 4-diméthylaminopyridine ou de pyridine.

Il va de soi que la réaction de Wittig, les clivages des fonctions esters, l'estérification sont des réactions bien connues de l'homme de métier et n'ont pas à être détaillées ici.

Les exemples suivants illustrent l'invention sans toutefois la limiter.

Exemple 1: 1R-cis-2,2-diméthyl-3-[(ΔZ)-2-fluoro-2-cyanoéthényl]cyclopropane-carboxylate de terbutyle et isomère ΔE correspondant.

On porte au reflux sous agitation pendant 1 heure un mélange renfermant 15 g de phénylbromo-fluoro-cyano-méthyl-mercure, 180 $cm^3$ de xylène, 8 g de triphénylphosphine et 5,25 g de 1R-cis-2,2-diméthyl-3-formyl-cyclopropane-1-carboxylate de terbutyle.

On glace, filtre et amène à sec le filtrat. On obtient 20 g d'un produit que l'on chromatographie sur silice en éluant par du benzène pur. On obtient:

3 g d'isomère ΔE (rf = 0,4)

1,3 g d'isomère ΔZ (rf = 0,33)

Préparation du phényl-bromo-fluoro-cyano-méthyl mercure utilisé au début de l'exemple.

a) Bromo-fluoro-acétamide

On ajoute à +5 °C, 86 $cm^3$ d'ammoniaque concentré dans 79 g de bromofluoro acétate d'éthyle en agitant fortement. On maintient encore 30 min l'agitation et la température, évapore à sec, distille le résidu et obtient 53,6 g de produit attendu; eb/0,1 = 82–84 °C.

b) Bromo-fluoro-acétonitrile

On ajoute 92 g d'anhydride phosphorique dans 183 g de bromo-fluoro-acétamide obtenu comme précédemment chauffé suffisamment pour mélanger, puis on chauffe progressivement le mélange jusqu'à 200 °C (extérieur) et distille entre 55 et 80 °C, 87 g de produit brut attendu.

c) Phényl-bromo-fluoro-cyano-méthyl-mercure

On refroidit à –50 °C, 15,65 g de chlorure de phényle mercurique dans 100 $cm^3$ de tétrahydrofuranne, ajoute 10,6 g de fluoro-bromo-acétonitrile obtenu comme ci-dessus, puis, toujours à –50 °C et sous agitation, une suspension contenant 7,85 g de terbutylate de potassium, 50 $cm^3$ de tétrahydrofuranne et 6,6 $cm^3$ d'alcool terbutylique.

Après 30 min, on verse le mélange réactionnel sur de l'eau glacée renfermant 6 $cm^3$ d'acide chlorhydrique concentré et extrait au chloroforme. On sèche la phase organique et concentre à sec. On reprend le résidu par un mélange chloroforme-hexane (1/1), filtre l'insoluble, glace, essore et obtient 9,3 g de produit attendu (F = 130–132 °C).

Exemple 2: Acide 1R-cis-2,2-diméthyl-3-[(ΔZ)-2-cyano-2-fluoroéthényl]cyclopropane-carboxylique.

On porte à 120–130 °C, 1,3 g de 1R-cis-2,2-diméthyl-3-[(ΔZ)-2-cyano-2-fluoroéthényl]cyclopropane-carboxylate de terbutyle, 13 $cm^3$ de méthylbenzène et 130 $cm^3$ d'acide paratoluènesulfonique. On maintient le mélange réactionnel 15 min sous agitation après le début du reflux. On ramène le mélange réactionnel à 20 °C, lave à l'eau, sèche sur sulfate de soude la solution toluénique, la filtre et l'amène à sec sous pression réduite. On obtient 1 g de produit recherché.

Exemple 3: Acide 1R-cis-2,2-diméthyl-3-[(ΔE)-2-cyano-2-fluoroéthényl]cyclopropane-carboxylique.

En opérant comme à l'exemple 4, à partir du 1R-cis-2,2-diméthyl-3-[(ΔE)-2-fluoro-2-cyanoéthényl-]cyclopropane-carboxylate de terbutyle, on obtient le produit recherché.

Exemple 4: 1R-cis-2,2-diméthyl-3-[(ΔZ)-2-fluoro-2-cyanoéthényl]cyclopropane-carboxylate de (S)-α-cyano-3-phénoxybenzyle.

On ajoute à 5 °C, 30 g de diméthylaminopyridine dans une solution renfermant 1 g d'acide 1R-cis-2,2-diméthyl-3-[(ΔZ)-2-fluoro-2-cyanoéthényl]cyclopropane-carboxylique, 6 $cm^3$ de chlorure de méthylène et 1,2 g d'alcool (S)-α-cyano-3-phénoxybenzylique. On ajoute ensuite 1,1 g de dicyclohexyl-carbodiimide et 8 $cm^3$ de chlorure de méthylène. On laisse le mélange réactionnel revenir à 20 °C et l'on maintient sous agitation pendant 2 h. On ajoute ensuite 0,5 $cm^3$ d'acide acétique et 0,5 $cm^3$ éthanol. On filtre ensuite l'urée formée, la rince avec un peu de chlorure de méthylène et amène, le filtrat à sec. On obtient 2,8 g de produit que l'on chromatographie sur silice en éluant par le mélange chlorure de méthylène, hexane (8/2). On obtient 1,5 g du produit recherché brut. Après recristallisation dans l'isopropanol au reflux, on obtient 1,3 g du produit recherché fondant à 90 °C.

Exemples 5 à 9:

En opérant comme précédemment, on a obtenu les produits répondant à la formule (I) suivants:

| Exemple | X | Δ | Géométrie | R |
|---|---|---|---|---|
| 5 | F | E | 1R cis | 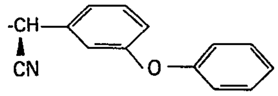 $\alpha_D=+57°5\pm3$ (c=0,4% $CHCl_3$) |
| 6 | F | E | 1R cis | 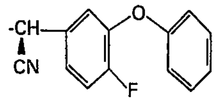 $\alpha_D=+86°\pm4°$ (c=0,2% $CHCl_3$) |
| 7 | F | E | 1R cis | 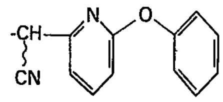 $\alpha_D=+61°\pm4°$ (c=0,2% $CHCl_3$) |
| 8 | F | Z | 1R cis | 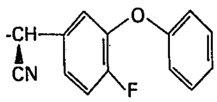 F=+124 °C |
| 9 | F | Z | 1R cis | 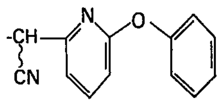 $\alpha_D=-31°5\pm4°$ (c=0,2% $CHCl_3$) |

C'est-à-dire:

le 1R-cis-2,2-diméthyl-3-[(ΔE)-2-fluoro-2-cyano-éthényl]cyclopropane-carboxylate de (S)α-cyano-3-phénoxybenzyle;

le 1R-cis-2,2-diméthyl-3-[(ΔE)-Z-fluoro-2-cyano-éthényl]cyclopropane-carboxylate de (S)α-cyano-3-phénoxy-4-fluorobenzyle;

le 1R-cis-2,2-diméthyl-3-[(ΔE)-2-fluoro-2-cyano-éthényl]cyclopropane-carboxylate de (R,S)-cyano--2-(6-phénoxypyridyl)-méthyle;

le 1R-cis-2,2-diméthyl-3-[(ΔZ)-2-fluoro-2-cyano-éthényl]cyclopropane-carboxylate de (S)α-cyano-3-phénoxy-4-fluorobenzyle;

le 1R-cis-2,2-diméthyl-3-[(ΔZ)-2-fluoro-2-cyano-éthényl]cyclopropane-carboxylate de (R,S)-cyano--2-(6-phénoxypyridyl)-méthyle.

Exemple 10: Préparation d'un concentré soluble.

On a effectué un mélange homogène de:

| | |
|---|---|
| produit de l'exemple 4 | 0,25 g |
| butoxyde de pipéronyle | 1,0 g |
| tween 80 | 0,25 g |
| topanol A | 0,1 g |
| eau | 98,4 g |

Exemple 11: Préparation d'un concentré émulsifiable

| | |
|---|---|
| produit de l'exemple 5 | 0,015 g |
| butoxyde de pipéronyle | 0,5 g |
| topanol A | 0,1 g |
| tween 80 | 3,5 g |
| xylène | 95,885 g |

Etude biologique des composes de l'invention.

A) Etude de l'effet d'abattage sur mouche domestique.

Les insectes tests sont des mouches domestiques femelles âgées de 4 jours. On opère par pulvérisation directe à la concentration de 0,25 g/l en chambre de Kearns et March en utilisant comme solvant un mélange d'acétone (5%) et d'Isopar L (solvant pétrolier) (quantité de solvant utilisée 2 ml en une seconde). On utilise 50 insectes par traitement. On effectue les contrôles toutes les minutes jusqu'à 10 min, puis à 15 min et l'on détermine le KT 50 par les méthodes habituelles.

| Composés des exemples | KT 50 en mn |
|---|---|
| 4 | 3,6 |
| 6 | 1,9 |
| 7 | 1,4 |
| 8 | 3,0 |
| 9 | 1,9 |

B) Etude de l'effet létal sur blatte.

Les tests sont effectués par contact sur film de verre, par dépôt à la pipette, de solutions acétoniques de différentes concentrations sur fond de boîte de Pétri en verre dont les bords ont été préalablement talqués afin d'éviter la fuite des insectes. On détermine la concentration létale 50 (CL 50), dose qui permet de tuer la moitié des blattes.

| Composés des exemples | CL 50 en $mg/m^2$ |
|---|---|
| 6 | 0,47 |
| 8 | 0,26 |

C) Etude de l'effet létal sur larves de Spodoptera littoralis.

Les essais sont effectués par application topique d'une solution acétonique toxique à l'aide du micromanipulateur d'Arnold sur le thorax dorsal des chenilles. On détermine la $DL_{50}$ du produit en

utilisant 15 larves par dose de produit à tester. Les chenilles utilisées sont des larves du quatrième stade larvaire, c'est-à-dire âgées d'environ 10 jours en élevage sur milieu artificiel (milieu de Poitout) à 24 °C et 65% d'humidité relative. Après traitement, les individus sont mis en observations sur milieu nutritif artificiel.

On effectue le contrôle des mortalités 48 h après traitement.

Les résultats expérimentaux obtenus pour les produits des exemples 4, 6 et 8 sont compris entre 0,48 et 4,8 ng par insecte.

**Revendications**

1. Sous toutes leurs formes isomères possibles ou sous forme de mélanges de ces isomères, les composés de formule (I):

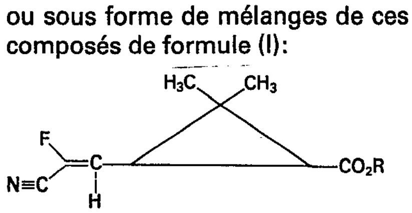 (I)

dans laquelle la copule acide cyclopropanique est de structure 1R cis la géométrie de la double liaison portée par la carbonne en 3 peut être de structure E ou Z et R représente un radical alcoyle linéaire, ramifié ou cyclisé, saturé ou insaturé, renfermant de 2 à 12 atomes de carbonne ou un radical choisi dans le groupe constitué par les radicaux suivants:

– les radicaux

-CH-Ar
|
Z

dans lesquels Z représente un radical éthynyle, méthyle ou cyano et Ar représente un radical $C_6H_5$, $C_6F_5$,

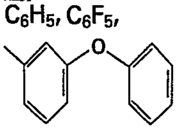, 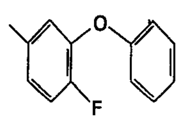,

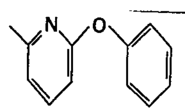, 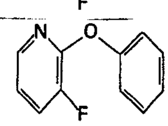 et

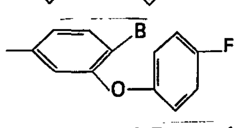

dans lequel B représente un atome d'hydrogène ou de fluor.

2. Les composés de formule (I) telle que définie à la revendication 1 dans laquelle la géométrie de la double liaison portée par le carbone en 3 du cyclopropane est du structure Z.

3. Les composés de formule (I) telle que définie à l'une quelconque des revendications 1 et 2 dans laquelle R représente un radical

-CH-Ar
|
Z

dans lequel Z représente un radical C≡CH, $CH_3$, un radical -C≡N et Ar représente un radical choisi dans le groupe des radicaux

$C_6H_5$

$C_6F_5$

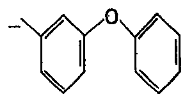 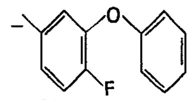

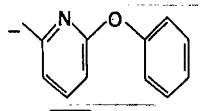, 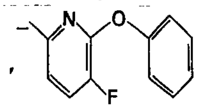

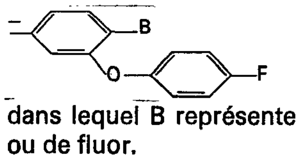

dans lequel B représente un atome d'hydrogène ou de fluor.

4. Les composés de formule (1) telle que définie à la revendication 3, dans laquelle Ar représente un radical α-cyano-3-phénoxybenzyle.

5. Les composés de formule (1) telle que définie à la revendication 1, dans laquelle R représente un radical

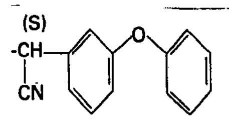 ou 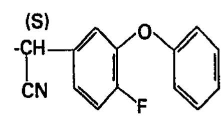

6. L'un quelconque des composés de formule (I), telle que définie à la revendication 1, dont les noms suivent:

– le 1R-cis-2,2-diméthyl-3-[ΔE-2-fluoro-2-cyanoéthényl]cyclopropane carboxylate de (S)α-cyano-3-phénoxybenzyle et l'isomère ΔZ correspondant;

– le 1R-cis-2,2-diméthyl-3-[ΔE-2-fluoro-2-cyanoéthényl]cyclopropane carboxylate de (S)α-cyano-3-phénoxy-3-fluorobenzyle et l'isomère ΔZ correspondant;

– le 1R-cis-2,2-diméthyl-3-[ΔE-2-fluoro-cyanoéthényl]-cyclopropane carboxylate de (R,S)-cyano-2-(6-phénoxypyridyl)-méthyle et l'isomère ΔZ correspondant.

7. Application des composés de formule (1) tels que définis à l'une quelconque des revendications 1 à 6 à la lutte contre les parasites des végétaux et les parasites des locaux.

8. Les compositions destinées à la lutte contre les parasites des végétaux, les parasites des locaux et les parasites des animaux à sang chaud, caractérisés en ce qu'elles renferment comme principe actif au moins un des produits définis à l'une quelconque des revendications 1 à 6.

9. Les compositions insecticides renfermant comme principe actif au moins l'un des produits définis à l'une quelconque des revendications 1 à 6.

10. Les compositions acaricides renfermant comme principe actif au moins l'un des produits définis à l'une quelconque des revendications 1 à 6.

11. Les compositions nématicides renfermant comme principe actif au moins l'un des produits définis à l'une quelconque des revendications 1 à 6.

12. Les compositions acaricides destinées à la lutte contre les parasites des animaux à sang chaud, notamment contre les tiques et les gales, caractérisées en ce qu'elles renferment comme principe actif l'un au moins des produits définis à l'une quelconques des revendications 1 à 6.

13. Les compositions destinées à l'alimentation animale renfermant comme principe actif au moins l'un des produits définis à l'une quelconque des revendications 1 à 6.

14. Les compositions selon l'une quelconque des revendications 10 à 16, caractérisées en ce qu'elles renferment en outre un synergiste des pyréthrinoïdes choisi dans le groupe des synergistes suivants: 1-(2,5,8-trioxadodécyl)-2-propyl-4,5-méthylène-dioxy-benzène (ou butoxyde de pipéronyle) ou le N-(2-éthylheptyl)-bicyclo-[2,2-1]5-heptène-2,3-dicarboximide ou le pipéronyl-bis-2-(2n-butoxyéthoxy)-éthyl acétal (ou tropital).

15. Procédé de préparation des composés de formule (I) tels que définis à l'une quelconque des revendications 1 à 6, caractérisé en ce que l'on soumet selon la réaction de Wittig un composé de formule (II):

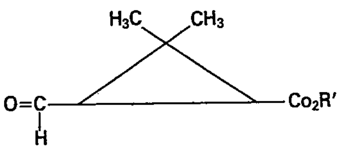
(II)

dans laquelle R' représente un atome d'hydrogène ou R, R conservant la même signification que dans la revendication 1, à l'action d'un composé de formule (III):

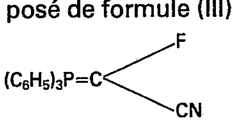
(III)

pour obtenir un composé correspondant, que l'on soumet à l'action d'un agent d'estérification si R' représente un atome d'hydrogène ou que l'on soumet, si désiré, à l'action d'un agent de clivage de la fonction ester, puis à l'action d'un agent d'estérification.

**Patentansprüche**

1. In sämtlichen ihrer möglichen isomeren Formen oder in Form von Gemischen dieser Isomeren, die Verbindungen der Formel (I):

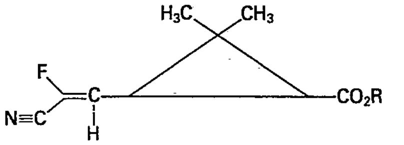
(I)

worin die Cyclopropansäureverknüpfung die 1R-cis-Struktur besitzt, die Geometrie der von dem Kohlenstoffatom in 3-Stellung getragenen Doppelbindung die E- oder Z-Struktur aufweisen kann und R einen linearen, verzweigten oder cyclischen gesättigten oder ungesättigten Alkylrest mit 2 bis 12 Kohlenstoffatomen oder einen Rest, ausgewählt unter den folgenden Resten bedeutet:

den Rest

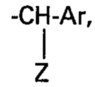

worin Z einen Ethynyl-, Methyl- oder Cyanorest bedeutet und Ar einen Rest

$C_6H_5$, $C_6F_5$,

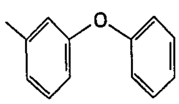
,
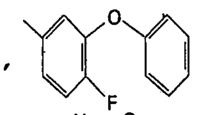
,
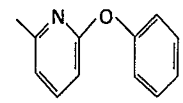
,
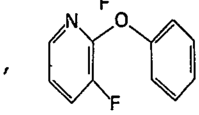

und 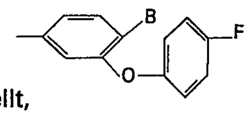

darstellt,

worin B ein Wasserstoff- oder Fluoratom bedeutet.

2. Die Verbindungen der Formel (I) gemäss Anspruch 1, worin die Geometrie der von dem Kohlenstoffatom in 3-Stellung des Cyclopropans getragenen Doppelbindung die Z-Struktur ist.

3. Verbindungen der Formel (I) gemäss einem der Ansprüche 1 und 2, worin R einen Rest

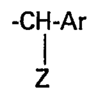

bedeutet, worin Z einen Rest -C≡CH, $-CH_3$, einen Rest -C≡N und Ar einen Rest, ausgewählt unter den folgenden Resten, bedeutet:

$C_6H_5$

$C_6F_5$

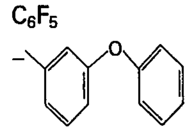
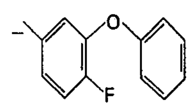
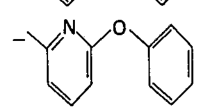
,
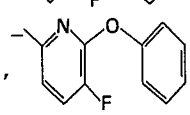
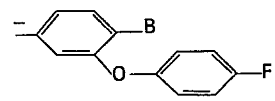

worin B ein Wasserstoff- oder Fluoratom darstellt.

4. Die Verbindungen der Formel (I) gemäss Anspruch 3, worin Ar einen α-Cyano-3-phenoxybenzylrest darstellt.

5. Die Verbindungen der Formel (I) gemäss Anspruch 1, worin R einen Rest

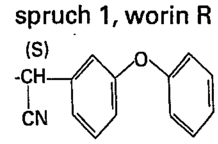
oder
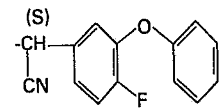

bedeutet.

6. Eine der Verbindungen der Formel (I) gemäss Anspruch 1 mit den folgenden Bezeichnungen:

(S)α-Cyano-3-phenoxybenzyl-1R-cis-2,2-dimethyl-3-[ΔE-2-fluoro-2-cyanoethenyl]-cyclopropancarboxylat und das entsprechende ΔZ-Isomere;

(S)α-Cyano-3-phenoxy-4-fluorobenzyl-1R-cis-2,2-dimethyl-3-[ΔE-2-fluoro-2-cyanoethenyl]-cy-

clopropancarboxylat und das entsprechende ΔZ-Isomere;

(R,S)-Cyano-2-(6-phenoxypyridyl)-methyl-1R-cis-2,2-dimethyl-3-[ΔE-2-fluoro-2-cyanoethenyl]-cyclopropancarboxylat und das entsprechende ΔZ-Isomere.

7. Verwendung der Verbindungen der Formel (I) gemäss einem der Ansprüche 1 bis 6 zur Bekämpfung von Parasiten der Pflanzen und Parasiten von Räumlichkeiten.

8. Zusammensetzungen für die Bekämpfung von Parasiten der Pflanzen, Parasiten von Räumlichkeiten und Parasiten warmblütiger Tiere, dadurch gekennzeichnet, dass sie als Wirkstoff zumindest eines der gemäss einem der Ansprüche 1 bis 6 definierten Produkte enthalten.

9. Insekticide Zusammensetzungen, enthaltend als Wirkstoff zumindest eines der Produkte gemäss einem der Ansprüche 1 bis 6.

10. Acaricide Zusammensetzungen, enthaltend als Wirkstoff zumindest eines der Produkte gemäss einem der Ansprüche 1 bis 6.

11. Nematicide Zusammensetzungen, enthaltend als Wirkstoff zumindest eines der Produkte gemäss einem der Ansprüche 1 bis 6.

12. Acaricide Zusammensetzungen für die Bekämpfung von Parasiten warmblütiger Tiere, insbesondere von Zecken und Krätzmilben, dadurch gekennzeichnet, dass sie als Wirkstoff zumindest eines der gemäss einem der Ansprüche 1 bis 6 definierten Produkte enthalten.

13. Zusammensetzungen für die tierische Ernährung, enthaltend als Wirkstoff zumindest eines der gemäss einem der Ansprüche 1 bis 6 definierten Produkte.

14. Zusammensetzungen gemäss einem der Ansprüche 10 bis 13, dadurch gekennzeichnet, dass sie ausserdem einen Synergisten der Pyrethrinoide, ausgewählt unter den folgenden Synergisten enthalten: 1-(2,5,8-Trioxadodecyl)-2-propyl-4,5-methylen-dioxybenzol (oder Piperonylbutoxyd) oder N-(2-Ethylheptyl)-bicyclo-[2,2-1]-5-hepten-2,3-dicarboximid oder Piperonyl-bis-2-(2n-butoxyethoxy)-ethylacetal (oder Tropital).

15. Verfahren zur Herstellung von Verbindungen der Formel (I) gemäss einem der Ansprüche 1 bis 6, dadurch gekennzeichnet, dass man eine Verbindung der Formel (II):

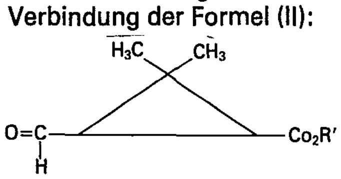 (II)

worin R′ ein Wasserstoffatom oder R bedeutet, wobei R die in Anspruch 1 angegebene Bedeutung besitzt, nach der Wittig-Reaktion der Einwirkung einer Verbindung der Formel (III):

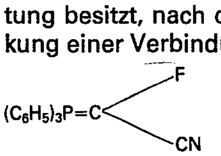 (III)

unterzieht, um eine entsprechende Verbindung zu erhalten, die man der Einwirkung eines Veresterungsmittels unterzieht, wenn R′ ein Wasserstoffatom bedeutet, oder gewünschtenfalls der Einwirkung eines Mittels zur Spaltung der Esterfunktion und danach der Einwirkung eines Veresterungsmittels unterzieht.

**Claims**

1. In all their possible isomeric forms or in the form of mixtures of these isomers, the compounds with the formula (I):

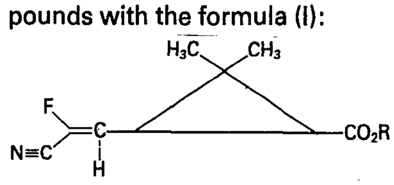 (I)

in which the cyclopropanic acid copula is of 1R cis structure, the geometry of the double bond carried by the carbon in position 3 can be of E or Z structure and R represents a linear, branched or cyclic alkyl radical, saturated or unsaturated, containing from 2 to 12 carbon atoms or a radical chosen from the group constituted by the following radicals:

the

-CH-Ar
|
Z

radicals in which Z represents an ethynyl, methyl or cyano radical and Ar represents a

$C_6H_5$, $C_6F_5$,

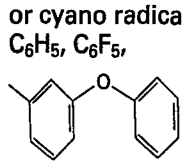, 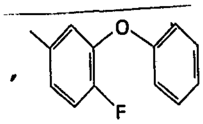,

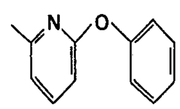, 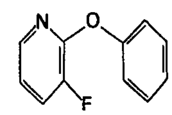

and 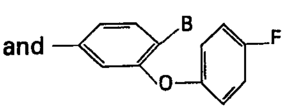

radical, in which B represents a hydrogen or fluorine atom.

2. The compounds with the formula (I) as defined in claim 1 in which the geometry of the double bond carried by the carbon in position 3 of the cyclopropane is of Z structure.

3. The compounds with the formula (1) as defined in any one of the claims 1 and 2 in which R represents a

-CH-Ar
|
Z

radical in which Z represents a $-C\equiv CH$, $-CH_3$ radical, a $-C\equiv N$ radical and Ar represents a radical chosen from the group of radicals

$C_6H_5$

$C_6F_5$

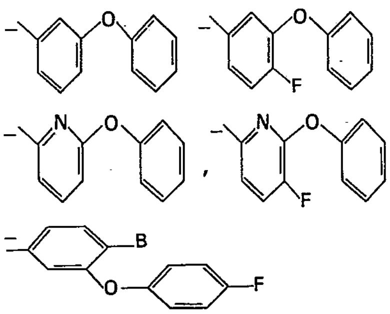

in which B represents a hydrogen or fluorine atom.

4. The compounds with the formula (I) as defined in claim 3, in which Ar represents a α-cyano-3-phenoxybenzyl radical.

5. The compounds with the formula (I) as defined in claim 1, in which R represents a

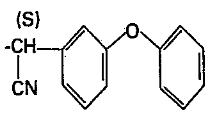 or a 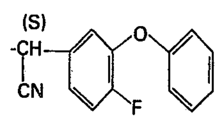

radical.

6. Any one of the compounds with the formula (I), as defined in claim 1, of which the names follow:

– 1R-cis-2,2-dimethyl-3-[ΔE-2-fluoro-2-cyanoethenyl]cyclopropanecarboxylate of (S)α-cyano-3-phenoxybenzyl and the corresponding ΔZ isomer;

– 1R-cis-2,2-dimethyl-3-[ΔE-2-fluoro-2-cyanoethenyl]cyclopropanecarboxylate of (S)α-cyano-3-phenoxy-4-fluorobenzyl and the corresponding ΔZ isomere;

– 1R-cis-2,2-dimethyl-3-[ΔE-2-fluoro-2-cyanoethenyl]cyclopropanecarboxylate of (R,S)-cyano-2-(6-phenoxypyridyl)methyl and the corresponding ΔZ isomer.

7. Use of the compounds with the formula (I) as defined in any one of the claims 1 to 6 for combatting parasites of vegetation and parasites of premises.

8. Compositions intended for combatting parasites of vegetation, parasites of premises and parasites of warm-blooded animals, characterized in that they contain as active principle at least one of the products defined in any one of the claims 1 to 6.

9. Insecticide compositions containing as active principle at least one of the products defined in any one of the claims 1 to 6.

10. Acaricide compositions containing as active principle at least one of the products defined in any one of the claims 1 to 6.

11. Nematocide compositions containing as active principle at least one of the products defined in any one of the claims 1 to 6.

12. Acaricide compositions intended for combatting parasites of warmblooded animals, particularly ticks and mites, characterized in that the contain as active principle at least one of the products defined in any one of the claims 1 to 6.

13. Compositions intended for animal fodder containing as active principle at least one of the products defined in any one of the claims 1 to 6.

14. Compositions according to any one of the claims 10 to 16, characterized in that they contain in addition a pyrethrinoid synergist chosen from the group of synergists which follow:

1-(2,5,8-trioxadodecyl)-2-propyl-4,5-methylenedioxybenzene (or piperonyl-butoxide) or N-(2-ethylheptyl)-bicyclo[2,2,1]-5-heptene-2,3-dicarboximide or piperonyl-bis-2-(2n-butoxyethoxy-)ethyl-acetal (or tropital).

15 Preparation process for compounds with the formula (I) as defined in any one of the claims 1 to 6, characterized in that a compound with the formula (II):

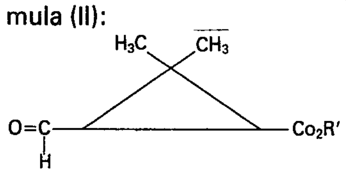 (II)

in which R′ represents a hydrogen atom or R, R retaining the same significance as in claim 1, is submitted according to Wittig's reaction to the action of a compound with the formula (III):

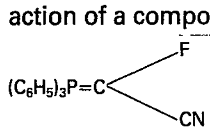 (III)

so as to obtain a corresponding compound, which is submitted to the action of an esterification agent if R′ represents a hydrogen atom, or which is submitted, if desired, to the action of a cleavage agent of the ester function, then to the action of an esterification agent.